(19) Europäisches Patentamt
European Patent Office
Office européen des brevets

(11) Veröffentlichungsnummer : **0 338 054 B1**

# (12) EUROPÄISCHE PATENTSCHRIFT

(45) Veröffentlichungstag der Patentschrift : **30.12.92 Patentblatt 92/53**

(21) Anmeldenummer : **88909206.0**

(22) Anmeldetag : **08.10.88**

(86) Internationale Anmeldenummer : **PCT/EP88/00901**

(87) Internationale Veröffentlichungsnummer : **WO 89/03812 05.05.89 Gazette 89/10**

(51) Int. Cl.$^5$ : **C07B 37/00,** C07C 25/00, C07C 13/00, C07C 43/20, C07C 49/807, C07C 255/50, C07D 213/60, C07D 239/24, // C09K19/00

## (54) KREUZKOPPLUNG VON TITANORGANYLEN.

Die Akte enthält technische Angaben, die nach dem Eingang der Anmeldung eingereicht wurden und die nicht in dieser Patentschrift enthalten sind.

(30) Priorität : **28.10.87 DE 3736489**

(43) Veröffentlichungstag der Anmeldung : **25.10.89 Patentblatt 89/43**

(45) Bekanntmachung des Hinweises auf die Patenterteilung : **30.12.92 Patentblatt 92/53**

(84) Benannte Vertragsstaaten : **CH DE GB LI**

(56) Entgegenhaltungen :
**DE-A- 3 237 367**
**J. Am. Chem. Soc. 109, 2393-2401 (1987)**
**CHEMICAL ABSTRACTS, vol. 102, Nr.15, 15 April 1985, (Columbus, Ohio, US), Tolstikov G.A. : "Cross-coupling reaction ofphenyltriethoxytitanium with allyl halides catalyzed by palladium complexes", see page 590, abstract 131596w**
**CHEMICAL ABSTRACTS, vol. 108, Nr. 5, 1 February 1988, (Columbus, Ohio, US), Kasatkin A.N. et al.: "Transition metalo-complexes in organic synthesis.I. Cross-coupling reactions of organotitanium compounds with allylic halides in the presence ofpalladium complexes", see pages 655-656, abstract 38014u**

(56) Entgegenhaltungen :
**CHEMICAL ABSTRACTS, vol. 94, Nr. 13, 30 March 1981, (Columbus, Ohio, US), Reetz M.T. et al.: "Efficient coupling of tertiaryalkyl halides with dialkylzinc and titanium compounds", see page 696, abstract 102895d**
**CHEMICAL ABSTRACTS, vol. 100, Nr. 19, 7 May 1984, (Columbus, Ohio, US), see page 511, abstract 156347N**
**CHEMICAL ABSTRACTS, vol. 106, Nr. 4, 26 January 1987, (Columbus, Ohio, US), see page 507, abstract 26206D**
**CHEMICAL ABSTRACTS, vol. 96, Nr. 7, 15 February 1982, (Columbus, Ohio, US), P. Skrabal et al.: "Substituted2-phenylnapthalenes, a new class of nematic liquid crystals.", see page 586, abstract 51939c**

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **POETSCH, Eike**
**Am Buchwald 4**
**W-6109 Mühltal (DE)**
Erfinder : **MEYER, Volker**
**Semder Pfad 23**
**W-6112 Gross Zimmern 2 (DE)**
Erfinder : **BÖTTCHER, Henning**
**Soderstrasse 95**
**W-6100 Darmstadt (DE)**

EP 0 338 054 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Verbindungen der Formel I,

$$R^1\text{-}A^o\text{-}(Z^1\text{-}A^1)_m\text{-}Z^o\text{-}(E)_n\text{-}Q\text{-}(Z^2\text{-}A^2)_p\text{-}R^2 \qquad I$$

worin

$R^1$ und $R^2$ jeweils unabhängig voneinander H oder Alkyl mit 1-18 C-Atomen, worin auch eine oder zwei nicht benachbarte $CH_2$-Gruppen durch -O-, -CO-, -O-CO-, -E-, -C≡C- und/oder -CO-O- ersetzt sein können, CN oder Halogen,

$A^o$, $A^1$ und $A^2$ jeweils unabhängig voneinander eine 1,4-Cyclohexylengruppe, worin auch eine oder zwei nicht benachbarte $CH_2$-Gruppen durch -O- und/oder -S- ersetzt sein können und/oder die in 1-Stellung durch $R^3$ substituiert sein kann, eine 1,4-Cyclohexenylen-, eine Piperidin-1,4-diyl- oder 1,4-Bicyclo-[2,2,2]-octylengruppe, eine unsubstituierte oder ein- oder zweimal durch $R^3$, NC, NCO, und/oder NCS substituierte 1,4-Phenylengruppe, worin auch eine oder mehrere CH-Gruppen durch N ersetzt sein können, oder eine Pyridin-3,5-diylgruppe,

E $CR^3$=$CR^4$ oder $CHR^3$-$CHR^4$,

$R^3$, $R^4$ jeweils unabhängig voneinander H, Alkyl mit 1-6 C-Atomen, F, Cl, Br, $CF_3$, CN, mit der Maßgabe, daß wenn E = $CHR^3$-$CHR^4$, $R^3$ und $R^4$ jeweils unabhängig voneinander Alkyl mit 1-6 C-Atomen, F, Cl, Br, $CF_3$ bedeuten,

$Z^o$ eine Einfachbindung oder Alkylen mit 1-4 C-Atomen, worin auch eine oder zwei $CH_2$-Gruppen durch -CH(CN)-, -CH(F)- oder -CH(Cl)-Gruppen ersetzt sein können,

$Z^1$ und $Z^2$ jeweils -CO-O-, -O-CO-, $-CH_2CH_2-$, $-CHCN-CH_2-$, $-CH_2-CHCN-$, -CH=CH-, $-OCH_2-$, $-CH_2O-$, -CH=N-, -N=CH-, -NO=N-, -N=NO- oder eine Einfachbindung,

Q eine unsubstituierte oder ein- zweimal durch $R^3$ substituierte 1,4-Phenylengruppe, worin auch eine oder mehrere CH-Gruppen durch N ersetzt sein können, oder eine Pyridin-3,5-diylgruppe

oder eine 1,4-Cyclohexylengruppe, worin auch eine oder zwei nicht benachbarte $CH_2$-Gruppen durch -O- und/oder -S- ersetzt sein können und/oder die in 1-Stellung durch $R^3$ substituiert sein kann, eine 1,4-Cyclohexenylen- oder 1,4-Bicyclo[2,2,2]-octylengruppe,

m und p jeweils 0, 1 oder 2,

n 0 oder 1 bedeuten,

mittels Titanorganylen.

Die bisher bekannten Verfahren zur Herstellung von Verbindungen der Formel I führen alle zu unbefriedigenden Ergebnissen. Hierfür sind vor allem schlechte Ausbeuten und z.Tschwer zugängliche Ausgangsverbindungen verantwortlich. So gelingt beispielsweise die Herstellung von 1-Cyano-2-fluor-4-(trans-4-heptylcyclohexyl)-benzol, einer Verbindung der Formel I, nach der EP-0 119 756 aus der entsprechenden 1-Acetylverbindung nur mit einer Ausbeute von ca. 1 % d.Th.

Auch die Herstellung von 1-Fluor-2-(trans-4-alkylcyclohexyl)-Verbindungen nach der in US-4 405 488 offenbarten Methode erfordert erheblichen synthetischen Aufwand und liefert die gewünschten Produkte nur in unbefriedigenden Ausbeuten.

Weiterhin sind Verfahren bekannt, die es erlauben, unter Übergangsmetallkatalyse Zink-, Zirkonium-, Aluminium-, Lithium-, Bor-, Quecksilber-, Kupfer- oder Cadmiumorganyle (z.B. DE-OS 36 32 410 oder Negishi et al., J. Am. Chem. Soc. 109, 2393 (1987)) mit Halogen-Verbindungen zu koppeln. Bei diesen Verfahren fallen regelmäßig schwer abzutrennende, die Abwässer belastende, toxische Übergangsmetallsalze an.

Darüberhinaus erzielen diese Verfahren keine vollständige Chemoselektivität bei polyfunktionellen Reaktionspartnern.

So greifen Grignard-Verbindungen und Lithiumorganyle unter Übergangsmetallkatalyse neben der C-Halogen-Bindung auch vorhandene Carbonylgruppen an oder erleiden eine oxidative Kopplung. Zink-Verbindungen reagieren auch mit α,β-ungesättigten Carbonylverbindungen (z.B. M. Luche et al., Tetrahedron Lett. 1984 3463) Alkenyltitan-Verbindungen sollen ebenfalls ungeeignet für Kreuzkopplungen sein (z.B. Negishi et al., J. Am. Chem. Soc. 109, 2393 (1987)).

In den Chemical Abstracts 102, 131596w und 108, 38014u werden Kreuzkopplungen von Organotitanverbindungen mit allylhalogeniden beschrieben, die, wie aus der Literatur bekannt, bei nucleophilen Substitutionsreaktionen äußerst reaktiv sind. Kreuzkopplungen mit anderen Halogeniden als Allylhalogeniden werden nich erwähnt.

Aufgabe der vorliegenden Erfindung war es, ein Herstellungsverfahren für die Verbindungen der Formel I zu finden, das die beschriebenen Nachteile der bisherigen Verfahren nicht oder nur in geringem Maß aufweisen.

Überraschenderweise wurde gefunden, daß titanorganische Verbindungen in Gegenwart eines Metallkatalysators hervorragend zur Herstellung von Verbindungen der Formel I geeignet sind.

Gegenstand der Erfindung ist somit ein Verfahren zur Herstellung von Verbindungen der Formel I, dadurch gekennzeichnet, daß man eine Organotitanverbindung der Formel II,

$$R^1\text{-}A^o\text{-}(Z^1\text{-}A^1)_m\text{-}Z^o\text{-}Ti(Y)_3 \qquad II$$

worin $R^1$, $A^o$, $A^1$, $Z^o$, $Z^1$ und m die für Formel I angegebene Bedeutung besitzen und Y jeweils unabhängig voneinander Cl, BR, $R^5$ O-$R^5$, $NR^5_2$ oder ein Rest $R^1$- $A^o$ -$(Z^1\text{-}A^1)_m$-$Z^0$- mit der voranstehenden Bedeutung ist, wobei $R^5$ verzweigtes oder unverzweigtes Alkyl mit 1-6 C-Atomen oder Cycloalkyl mit 3-7 C-Atomen bedeutet, mit einer Verbindung der Formel III,

$$X\text{-}(E)_n\text{-}Q\text{-}(Z^2\text{-}A^2)_p\text{-}R^2 \qquad III$$

worin $R^2$, E, $A^2$, $Z^2$, Q, n und p die für Formel I angegebene Bedeutung besitzen und X Br, J, $OSO_2\text{-}(CF_2)_r\text{-}CF_3$, worin r 0, 1, 2, 3, 4, 5, 6, 7, 8, 9 oder 10 ist, bedeutet, unter Zusatz einer Verbindung des Palladiums und/oder Nickels koppelt.

Die Verbindungen der Formel I sind teilweise bekannt, teilweise aber auch neu. Die neuen Verbindungen der Formel I sind ebenfalls Gegenstand der Erfindung.

Das Gelingen einer derartigen stereoselektiven Kopplung aliphatischer bzw. aromatischer Titanorganyle mit aromatischen, heteroaromatischen, vinylischen, aliphatischen oder cycloaliphatischen Halogenverbindungen oder Sulfonaten war um so überraschender, als bei bisher bekannten C-C-Kopplungsreaktionen eine β-Eliminierung aus der der Kopplungsposition benachbarten CH-Gruppe entweder bei der Herstellung des Metallorganyls oder aus dem Metallorganyl selbst als den sterischen Verlauf und die Ausbeute stark beeinflussende Nebenreaktion auftritt.

Weiterhin erweist sich das Verfahren als hochchemoselektiv, so daß selbst elektrophile Gruppen wie Ester oder Nitrile den Verlauf der Reaktion nicht beeinträchtigen können. Darüberhinaus läßt sich das nach Beendigung der Reaktion anfallende Titandioxid leicht abtrennen, so daß keinerlei belastende Stoffe in die Abwässer gelangen können. Auch lassen sich die Titanliganden Y beliebig variieren, wodurch die Chemo-, Stereo- und Enantioselektivitäten der Verbindungen der Formel II gezielt beeinflußt und den jeweiligen Bedingungen angepaßt werden können. Das erfindungsgemäße Verfahren geht von den leicht zugänglichen, metallierbaren Verbindungen der Formel IV aus

$$R^1\text{-}A^o\text{-}(Z^1\text{-}A^1)_m\text{-}Z^o\text{-}X' \qquad IV$$

worin $R^1$, $A^o$, $A^1$, $Z^o$, $Z^1$ und m die für Formel I angegebene Bedeutung besitzen und X' Chlor, Brom, Iod bedeutet.

Diese werden nach bekannten Verfahren, wie sie z.B. bei M.T. Reetz, Top. Curr. Chem. 106, 1 (1982) beschrieben sind, in die entsprechende Titanorganyle der Formel II überführt. Die angewandten Methoden werden dabei zweckmäßig der Natur der in den metallierbaren Verbindungen der Formel IV befindlichen funktionellen Gruppen angepaßt.

Die Umsetzung der metallierbaren Verbindungen der Formel IV (X' = Chlor, Brom, Jod) erfolgt vorzugsweise in einem inerten Lösungsmittel, beispielsweise Ethern wie Diethylether, Dimethoxyethan, Tetrahydrofuran oder Dioxan, Kohlenwasserstoffen wie Hexan, Cyclohexan, Benzol oder Toluol oder Gemischen dieser Lösungsmittel mit metallischen Lithium, n-Butyllithium, tert-Butyllithium, Lithiumnaphthalenid, Lithium-di-tert-Butylnaphthalenid oder metallischen Magnesium bei Temperaturen von -100° bis +100 °C vorzugsweise bei -78° bis +75 °C.

Unter diesen Bedingungen ist die Umwandlung der metallierbaren Verbindungen der Formel IV in die Lithium- oder Halogenmagnesiumorganyle (X' = Li oder MgX) gewöhnlich in etwa 10 bis 60 Minuten beendet.

Durch Zugabe von Titanverbindungen der Formel V,

$$Ti(Y')_4 \qquad V$$

worin Y' jeweils unabhängig voneinander Cl, Br, $R^5$, $OR^5$ oder $NR^5_2$ bedeutet, wobei $R^5$ die angegebene Bedeutung besitzt, zu den Lösungen der Halogenmagnesium- oder Lithiumorganylen bei Temperaturen von -78 °C bis 20 °C bevorzugt bei -30 °C bis 0 °C, erhält man die Titanorganyle in der Regel nach 60 bis 180 Minuten Reaktionsdauer.

Die Titanverbindungen der Formel V können gelöst oder ungelöst zugegeben werden. In der Regel werden sie gelöst zugegeben, bevorzugt im selben Medium, in dem das Halogenmagnesium- oder Lithiumorganyl vorliegt.

Anschließend fügt man der Lösung des Titanorganyls der Formel II die Kopplungskomponente der Formel III und einen geeigneten Metallkatalysator zu, wobei eine selektive Kopplung zu den Verbindungen der Formel I erfolgt.

Zweckmäßigerweise wendet man dabei die gleichen wie für die Herstellung der Titanorganyle der Formel II voranstehend genannten Lösungsmittel an bei den angegebenen Temperaturen. Die erforderlichen Reaktionszeiten betragen in der Regel zwischen etwa 1 und 50 Stunden.

Als Ausgangsverbindungen werden metallierbare Verbindungen der Formel IV und Verbindungen der Formel III eingesetzt. Als einsetzbare Halogenverbindungen kommen alle Halogenide, die sich in ein Titanorganyl

umwandeln lassen, in Frage. Vorzugsweise werden die Bromide eingesetzt, ferner auch die Chloride oder Iodide.

Die nachfolgenden Formeln IV b bis IVk stellen eine Gruppe von ganz besonders bevorzugten Ausgangsverbindungen dar, wobei die aromatischen Gruppen auch durch F, Cl, CN oder $CH_3$, die Cyclohexylgruppen auch in 1-Stellung durch F, Cl, $CF_3$, CN oder $C_1$-$C_4$ Alkyl substituiert sein können:

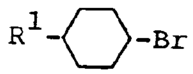

IVb

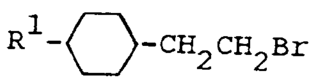

IVd

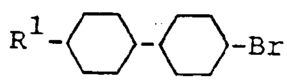

IVe

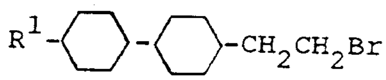

IVg

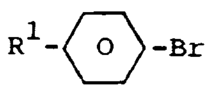

IVh

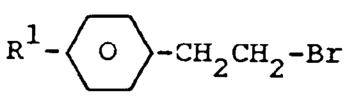

IVi

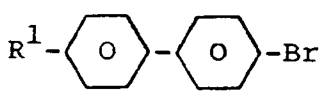

IVk

$R^1$ bedeutet darin vorzugsweise eine Alkylgruppe mit 1-18 C-Atomen, worin auch eine oder zwei nicht benachbarte $CH_2$-Gruppen durch -O- und/oder -CO- und/oder -CO-O-ersetzt sein können.

In den Formeln I, II, III und IV bedeuten $R^1$ bzw. $R^2$ vorzugsweise geradkettiges oder verzweigtes Alkyl mit 1-18 C-Atomen, vorzugsweise mit 2-10 C-Atomen, und bedeuten demnach bevorzugt Ethyl, Propyl, Butyl, Pentyl, Hexyl, Heptyl, Octyl, Nonyl oder Decyl, ferner auch Methyl, Undecyl, Dodecyl, Tridecyl, Tetradecyl oder Pentadecyl. Bevorzugt sind auch verzweigte Reste wie Isopropyl, 2-Butyl, Isopentyl, 1-Methylpentyl, 2-Methylpentyl, 1-Methylhexyl oder 1-Methylheptyl. $R^1$ bzw. $R^2$ bedeutet vorzugsweise auch Ethoxy, Propoxy, Butoxy, Pentoxy, Hexoxy, Heptoxy, Octoxy, Nonoxy und Decoxy, ferner auch Methoxy, Undecoxy, Dodecoxy, Tridecoxy, Tetradecoxy oder Pentadecoxy oder auch Isopropoxy, Isopentoxy, 2-Butoxy oder 1-Methylpentoxy.

Für die Kopplung mit den Titanorganylen der Formel II verwendbar sind alle Verbindungen der Formel III. Vorzugsweise werden die Bromide (X = Br) eingesetzt, und Perfluoralkansulfonate (X = $OSO_2$-$(CF_2)_r$-$CF_3$). Unter den Perfluoralkansulfonaten besonders bevorzugt sind die Trifluormethan-, Perfluorethan- und Perfluorpropansulfonate. Zur Kopplung von Titanorganylen mit verzweigten aliphatischen Verbindungen der Formel III sind besonders Jodide geeignet (X = J).

Die nachfolgenden Formeln III b bis IIIu stellen eine Gruppe von ganz besonders bevorzugten Ausgangsverbindungen dar, wobei die aromatischen Gruppen auch durch F, Cl, CN oder $CH_3$, die Cyclohexylgruppen auch in 1-Stellung durch F, Cl, $CF_3$, CN oder $C_1$-$C_4$ Alkyl substituiert sein können:

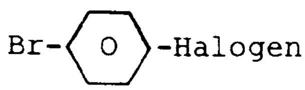 IIIb

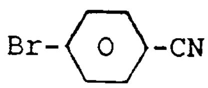 IIIc

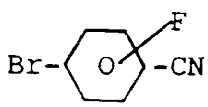 IIId

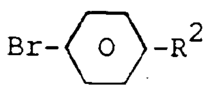 IIIe

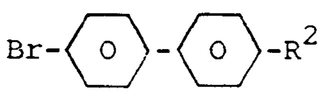 IIIf

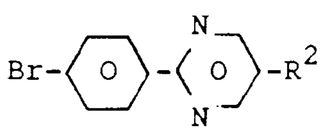 IIIg

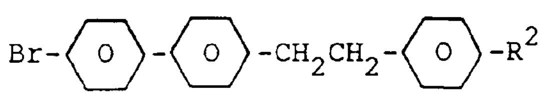 IIIh

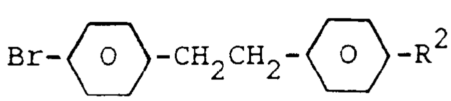 IIIi

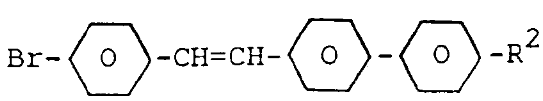 IIIj

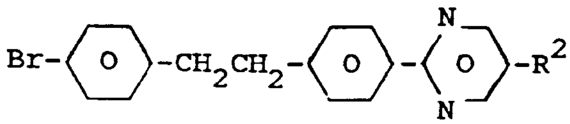

IIIk

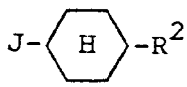

IIIm

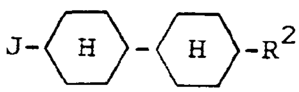

IIIn

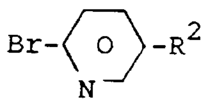

IIIp

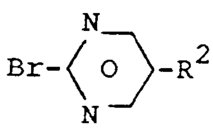

IIIq

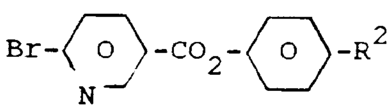

IIIr

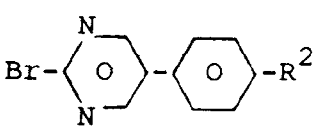

IIIs

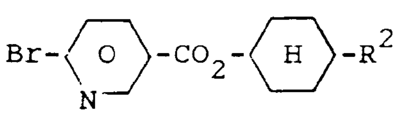

IIIt

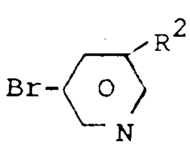

IIIu

$R^2$ bedeutet darin vorzugsweise eine Alkylgruppe mit 1-15 C-Atomen, worin auch eine oder zwei nicht benachbarte $CH_2$-Gruppen durch -O- und/oder -CO- und/oder -CO-O- ersetzt sein können.

Als Titanverbindung der Formel V einen sich besonders solche, die 4 gleiche Substituenten Y′ tragen, wie Titantetrachlorid und Titantetrabromid oder solche, die sowohl Halogen-Substituenten als auch Alkoxy- oder Dialkylamino-Gruppen enthalten, wie z.B. Verbindungen der Formel Va-Vn

| | |
|---|---|
| $Cl_3Ti(OR^5)$ | Va |
| $Cl_3Ti(NR^5_2)$ | Vb |
| $Br_3Ti(OR^5)$ | Vc |
| $Br_3Ti(NR^5_2)$ | Vd |
| $Cl_2Ti(OR^5)_2$ | Ve |
| $Cl_2Ti(NR^5_2)_2$ | Vf |
| $Br_2Ti(OR^5)_2$ | Vg |
| $Br_2Ti(NR^5_2)_2$ | Vh |
| $ClTi(OR^5)_3$ | Vi |
| $ClTi(NR^5_2)_3$ | Vj |
| $BrTi(OR^5)_3$ | Vk |
| $BrTi(NR^5_2)_3$ | Vl |
| $Cl_2Ti(R^5)_2$ | Vm |
| $Br_2Ti(R^5)_2$ | Vn |

$R^5$ bedeutet vorzugsweise eine Alkylgruppe mit 1-3 C-Atomen.

Besonders bevorzugt sind die Titanverbindungen Vi ($R^5$ = i-$C_3H_7$), Vl ($R^5$ = $CH_3$, $C_2H_5$) und Vm ($R^5$ = Cyclopentadienyl).

Als metallische Katalysatoren werden vorzugsweise solche des Palladiums und/oder Nickels verwendet. Zur Bildung geeigneter Komplexe befähigte Liganden finden sich beispielsweise in der Gruppe der Phosphine wie Triphenylphosphin, Tritolylphosphin, Tris-(4-dimethylaminophenyl)-phosphin oder 1,2-Bis-(diphenylphosphino)-ethan oder 1,1′-Bis-(diphenylphosphino)-ferrocen oder der Diketone wie Acetylaceton oder Octafluoracetylaceton.

Es können auch Salze der vorstehend genannten Metalle Verwendung finden, wie beispielsweise $LiPdCl_3$ der entsprechende Nickelsalze. Weiterhin eignen sich auch gemischte Komplexe wie Bis-(triphenylphosphin)-nickeldichlorid.

Die Verwendung von Tetrakis-(triphenylphosphin)-palladium oder von [1,1′-Bis-(Diphenylphosphin)ferrocenyl]-palladium als Katalysator stellt eine besonders bevorzugte Ausführungsform der vorliegenden Erfindung dar.

Die als Ausgangsverbindungen eingesetzten Halogenverbindungen der Formel IV sind bekannt oder können nach bekannten Methoden, wie beispielsweise in Houben-Weyl, Methoden der Organischen Chemie, Bd. 5/3 und 5/4 beschrieben, hergestellt werden. So ist es beispielsweise möglich, Alkohole der Formel IV, in denen X′ für eine Hydroxylgruppe steht, mit einem Phosphortrihalogenid oder mit Triphenylphosphin/Tetrahalogenmethan in die entsprechenden Halogenide zu überführen.

Die als Transmetallierungsreagenzien eingesetzten Titanverbindungen der Formel V sind bekannt oder können nach bekannten Methoden z.B. Gmelin Handbuch, Ti-Organische Verbindungen, 8. Auflage Band 40, N.Y. 1977 hergestellt werden.

Ebenfalls bekannt oder nach bekannten Methoden erhältlich sind die als Kopplungskomponenten einzusetzenden Verbindungen der Formel III.

Beispielsweise lassen sich Halogenide der Formel III, worin X Br oder I, n 0 und Q einen Aromaten bedeutet, dadurch erhalten, daß man in einem entsprechenden Diazoniumsalz die Diazoniumgruppe durch Br oder I ersetzt.

Perfluoralkylsulfonate der Formel III, worin X $OSO_2$-$(CF_2)_r$-$CF_3$ bedeutet, können durch Veresterung entsprechender Alkohole der Formel III, worin X eine Hydroxylgruppe bedeutet, mit Perfluoralkansulfonsäuren oder ihren reaktiven Derivaten hergestellt werden. Die entsprechenden Perfluoralkansulfonsäuren sind bekannt.

Als reaktionsfähige Derivate der genannten Perfluoralkansulfonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

In der vorliegenden Erfindung steht somit ein sehr vorteilhaftes Verfahren zur einfachen, in hohen Ausbeuten verlaufenden, stereoselektiven Herstellung von Verbindungen der Formel I zu Verfügung.

Die Verbindungen der Formel I eignen sich zum Einsatz als flüssigkristalline Materialien, wie z.B. in DE-32 17 597 und DE-26 36 684 offenbart, oder können als Zwischenprodukte für die Herstellung weiterer flüssigkristalliner Verbindungen verwendet werden.

Die folgenden Beispiele sollen die Erfindung näher erläutern. Fp = Schmelzpunkt; K = kristallin; N = nematisch, I = isotrop. Vor- und nachstehend bedeuten Prozentangaben Gewichtsprozent.

Übliche Aufarbeitung bedeutet im folgenden: Mit schwach saurem Wasser versetzen, mit Toluol extrahieren, Trocknen der organischen Phase, Eindampfen und Reinigen durch Chromatographie und/oder Kristallisation.

Beispiel 1

Ein Gemisch aus 1-Brommagnesium-4-pentyl-benzol (hergestellt aus 14,8 g 4-Pentyl-brombenzol und 1,6 g Magnesium) und 15 ml Tetrahydrofuran wird bei 0°-10 °C mit einer Lösung von 17,1 g Triisopropoxytitanchlorid in 50 ml Tetrafuran versetzt. Nach 30minütigem Rühren setzt man 14,5 g 3-Brom-6-octyloxypyridin und 0,53 g [1,2-Bis-(diphenylphosphino)ethano]-Nickelchlorid in 50 ml Tetrahydrofuran zu. Nach 16stündigem Rühren bei Raumtemperatur und üblicher Aufarbeitung erhält man 3-(4-Pentylphenyl)-6-octyloxypyridin von Schmelzpunkt 36 °C und Klärpunkt 42 °C.

Analog erhält man:

3-(4-Benzoyloxyphenyl)-6-octyloxypyridin K 106 I

Beispiel 2

5,75 g cis-4-(trans-4′-Propylcyclohexyl)-cyclohexylbromid (hergestellt aus dem entsprechenden trans-Alkohol mit Triphenylphosphin/Brom in Acetonitril in für die Bromierung von Alkoholen bekannter Weise) werden in einem Lösungsmittelgemisch von 50 ml Toluol/Tetrahydrofuran (4:1) mit 5,21 g Triisopropoxytitanchlorid und 0,28 g dünn gehämmerten Lithiumscheiben in einem gekühlten Ultraschallbad dem Ultraschall ausgesetzt bis kein Lithium erkennbar ist. Die Reaktionsmischung wird mit 0,16 g Tetrakis(triphenylphosphin)palladium(0) und 4 g 4-Brom-2-fluorbenzonitril versetzt und 18 h bei Zimmertemperatur gerührt. Darauf wird mit schwach saurem Wasser und Toluol extrahiert, die organische Phase über $MgSO_4$ getrocknet und eingedampft. Der Eindampfrückstand ergibt nach Chromatographie über Kieselgel mit Petrolether/Diethylether 9:1 und Kristallisation aus Ethanol 3,5 g 4-Cyano-3-fluor-1-[trans-4′-(trans-4″-propylcyclohexyl)-cyclohexyl]benzol vom Schmelzpunkt 54,3 °C und Klärpunkt 203 °C.

Auf analoge Weise erhält man aus 6,06 g (0,02 Mol) cis-4-(trans-4′-Butylcyclohexyl)-cyclohexylbromid und 5,4 g (0,02 Mol) 4-Trifluormethansulfonyloxy-2-fluorbenzonitril 2,3 g 4-Cyano-3-fluor-1-[trans-4′-(trans-4″-butylcyclohexyl)-cyclohexyl]-benzol; K/N 67°, N/I 197,4°.

Analog lassen sich die folgenden Verbindungen darstellen (die angegebenen Übergangstemperaturen beziehen sich auf den Übergang kristallin/nematisch (K/N) und nematisch/isotrop (N/I). 4-[trans-4′-(trans-4″-Alkylcyclohexyl)-cyclohexyl]-halogenbenzole

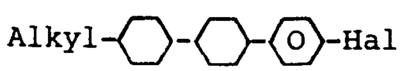

| Alkyl | Hal | K/N (°C) | N/I (°C) |
|---|---|---|---|
| $H_7C_3$ | F | 87,9 | 158,5 |
| $H_9C_4$ | F | 79,5 | 152,1 |
| $H_{13}C_6$ | Cl | 57,8 | 173,2 |

4-[trans-4′-(trans-4″-Alkylcyclohexyl)-cyclohexyl)-alkoxybenzole und -alkylbenzole

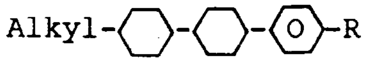

| Alkyl | R | K/N (°C) | N/I (°C) |
|---|---|---|---|
| $H_7C_3$ | $OCH_3$ | 79,5 | 211 |
| $H_9C_4$ | $OC_6H_{13}$ | 70,0 | 186,3 |
| $H_7C_3$ | $CH_3$ | 64,6 | 179,8 |
| $H_{11}C_5$ | $C_3H_7$ | 48,6 | 181,0 |
| $H_7C_3$ | $C_5H_{11}$ | < 20 | 172,0 |

4-[trans-4'-(trans-4"-Alkylcyclohexyl)-cyclohexyl]-halogenbenzole

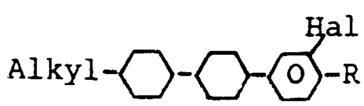

| Alkyl | Hal | R | K/N (°C) | N/I (°C) |
|---|---|---|---|---|
| $H_7C_3$ | F | H | 54,5 | 92,3 |
| $H_9C_4$ | Cl | H | 28,3 | 39,8 |
| $H_7C_3$ | F | $COCH_3$ | 88 | 293 |
| $H_9C_4$ | F | CN | 67 | 197,4 |

trans-4-Alkylcyclohexylarylderivate

Alkyl-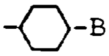-B-R

| Alkyl | B | R | K/N (°C) | N/I (°C) |
|---|---|---|---|---|
| $H_7C_3$ | 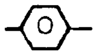 | 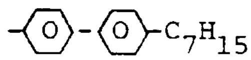 | | |
| $H_7C_3$ | 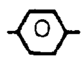 | 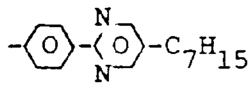 | 106 | 169 |
| $H_7C_3$ | 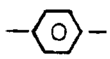 | 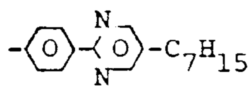 | | |
| $H_7C_3$ | 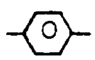 | 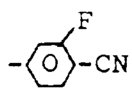 | | |

| | | | | |
|---|---|---|---|---|
| $H_9C_4$ | 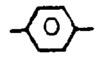 | 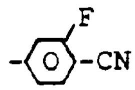 | | |
| $H_{11}C_5$ | 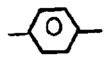 | 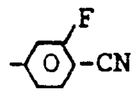 | | |
| $H_7C_3$ | 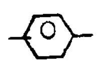 | 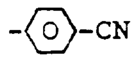 | | |
| $H_{15}C_7$ | 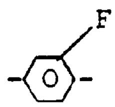 | -CN | 19 | 21 |
| $H_7C_3$ | 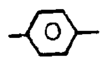 | 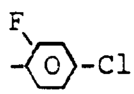 | 04 | 09 |
| $H_{11}C_5$ | 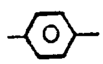 | 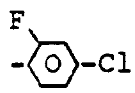 | 57,5 | 85 |
| $H_{15}C_7$ | 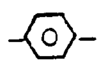 | 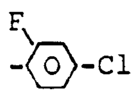 | 58,5 | 83 |
| $H_7C_3$ | 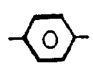 | 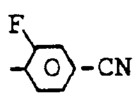 | 90 | 115,5 |
| $H_{11}C_5$ | 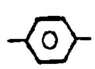 | 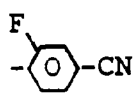 | 77,5 | 119,5 |
| $H_{11}C_5$ | 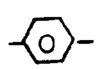 | 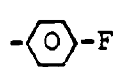 | 57 | 79 |
| $H_{11}C_5$ | 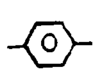 | 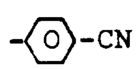 | 96 | 131 |

4-[trans-4′-(trans-4″-Alkylcyclohexyl)-cyclohexyl]-arylderivate

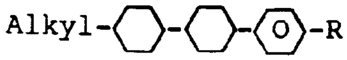

| Alkyl | R | K/N (°C) | N/I (°C) |
|---|---|---|---|
| $C_3H_7$ | $COCH_3$ | 81 | 115 |
| $C_2H_5$ | CN | 76 | 195 |
| $C_3H_7$ | CN | 55 | 184 |
| $C_5H_{11}$ | 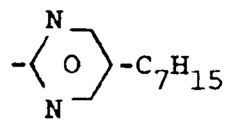 | 200 | 270 |

Beispiel 3

4,68 g trans-4-(trans-4′-Pentylcyclohexyl)-cyclohexancarbonsäurenitril werden in 7,5 ml Tetrahydrofuran gelöst und bei -75° bis -65 °C zu einer Lösung von Lithiumdiisopropylamid getropft, die in 7,5 ml Tetrahydrofuran aus 2,7 ml Diisopropylamin und 12,5 ml 15 %iger Hexanlösung von Butyllithium bereitet wird. Nach der Zugabe wird noch 30 Minuten nachgerührt, dann werden unter weiterem Rühren bei -78 °C 4,65 g Triisopropoxytitanchlorid gelöst in 10 ml Tetrahydrofuran, zugetropft. Es wird bei -50 °C eine halbe Stunde nachgerührt, dann werden 50 mg 1,2-Bis-diphenylphosphinoethano-Nickeldichlorid und 3,27 g 4-Brombenzonitril, gelöst in 15 ml Tetrahydrofuran, zugesetzt und nach Entfernung der Kühlung 72 Stunden bei Zimmertemperatur gerührt. Darauf wird mit Eiswasser Versetzt, eingedampft, mit Wasser/Petrolether extrahiert, die Petroletherphase getrocknet, eingedampft und über Kieselgel mit Petrolether/Diethylether 9:1 chromatographiert. Die Hauptfraktion ergibt nach zweimaliger Umkristallisation aus Essigsäureethylester 0,92 g 4-[cis-4-(trans-4- Pentylcyclohexyl)-r-1-cyanocyclohexyl]-benzonitril. Die Verwendung von $NiCl_2$ $(PPh_3)_2$ unter sonst gleichen Bedingungen führt zu vergleichbaren Ausbeuten.

Schmelzpunkt 113,3 °C, Klärpunkt: 135,6 °C, $\Delta\varepsilon$ = 2,3

Analog werden aus dem Titanderivat des trans-4-(trans-4′-Pentylcyclohexyl)-cyclohexancarbonsäurenitrils unter Verwendung des Dicyclopentadienyltitandichlorid erhalten:

| | Schmelzpunkt °C | Klärpunkt °C | $\Delta\varepsilon$ |
|---|---|---|---|
| 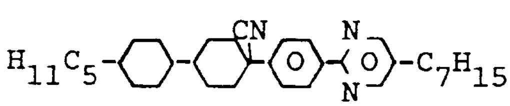 | 85,1 | 211,2 | -0,3 |
| 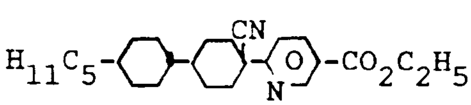 | 86,0 | 116,2 | -6,6 |

Beispiel 4

a) Vergleichsbespiel

5,75 g cis-4-(trans-4′-Propylcyclohexyl)-cyclohexylbromid werden mit 5,3 g Chlortriisopropoxytitan

analog Beispiel 2 in die Titanverbindung übergeführt. Es werden 0,16 g Tetrakis (triphenylphosphin)palladium(0) zugegeben und 3,2 g Vinylbromid zugetropft. Darauf wird 72 Stunden bei 30° gerührt, extraktiv mit Wasser und Toluol aufgearbeitet und der Toluoleindampfrückstand mit Naphtabenzin über Kieselgel filtriert. Nach dem Eindampfen werden 3,1 g eines Gemisches erhalten, das gemäß gaschromatograhischer Analyse zu über 40 % aus 1-[trans-4'-(Propylcyclohexyl)-trans-4"-cyclohexyl]-ethylen besteht.

b) Analog a)erhält man nach anschließender Umkristallisation durch Umsetzung mit den entsprechenden Bromstyrolderivaten, deren Herstellung nach den in Houben-Weyl Band V/4 angegebenen Methoden gelingt, die nachstehend aufgeführten 1-Aryl-2[trans-4'-(alkylcyclohexyl)-trans-4"-cyclohexyl]-ethene:

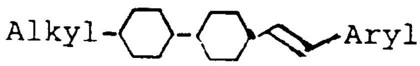

| Alkyl | Aryl | Schmelzpunkt | Klärpunkt |
|---|---|---|---|
| $C_4H_9$ | 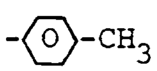 | 73 | 224 |
| $C_3H_7$ | 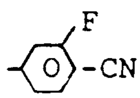 | 159 °C | 216 °C |
| $C_4H_9$ | 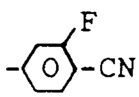 | 57 | 226 |

Beispiel 5

Eine Lösung von 20,5 g eines 1:1 Gemisches der isomeren 4-Propylcyclohexylbromide in 100 ml Tetrahydrofuran wird bei -78 °C zu 1,2 1 einer Lithium-di-tert-butylnaphthalin haltigen Tetrahydrofuranlösung gegeben (hergestellt nach K. Freeman, L. Hutchinson, J. Org. Chem. 45, 1924 (1980), aus 1,4 g Lithium und 48 g Di-tert-butylnaphthalin). Nach 5minütigem Rühren werden 26,0 g Triisopropoxytitanchlorid zugetropft. Nach 60minütigem Rühren bei -30 °C erhält man die 4-Propylcyclohexyltriisopropoxytitanverbindung. Diese ergibt in einer zu Beispiel 1 analogen Kopplungsreaktion mit 18,2 g Brombenzonitril 7,5 g 4-(trans-4-propylcyclohexyl)-benzonitril.

Analog werden die folgenden Verbindungen aus 4-substituierten Brombenzol-Derivaten erhalten:

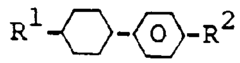

| $R^1$ | $R^2$ | Schmelzpunkt °C | Klärpunkt °C |
|---|---|---|---|
| Propyl | F | 32 | |
| Heptyl | F | 35 | |
| Heptyl | Propyloxy | 46 | |
| Pentyl | Butyloxy | 34 | 46 |
| Pentyl | Hexyloxy | 33 | 45 |
| Heptyl | Hexyloxy | 38 | 52 |

Beispiel 6

Ein Gemisch aus 1-Propylmagnesiumbromid, hergestellt aus 46,5 g 1-Brompropan und 13,4 g Magnesiumspänen, und 200 ml Diethylether, wird mit 66,5 ml 1-Vinylcyclohex-3-en tropfenweise versetzt.

Nach Zugabe von 1,45 ml Titantetrachlorid, Erhitzen und Abkühlen gibt man ein Gemisch aus 92,8 g 4-Brombenzonitril, 5,8 g [1,1'-Bis-(Diphenylphosphin)ferrocenyl]-palladium(O), erhalten nach Beispiel 8, 300 ml Diethylether und 200 ml Tetrahydrofuran. Nach üblichem Aufarbeiten erhält man 4-(2-Cyclohex-3-enylethyl)benzonitril.

Beispiel 7

Zu einem Gemisch aus Brommagnesiumbenzol (hergestellt aus 14 ml Brombenzol und 3,2 g Magnesiumspänen) und 75 ml Tetrahydrofuran gibt man bei 10°-20 °C eine Lösung von 34,2 g Triisopropoxytitanchlorid in 50 ml Tetrahydrofuran. Nach 30minütigem Rühren tropft man 1,3 g Bistriphenylphosphino-Nickel-(II)-chlorid und 21,6 g 5-Bromnikotinsäuremethylester in 150 ml Tetrahydrofuran hinzu. Nach 24stündigem Rühren und üblichem Aufarbeiten und chromatographischer Trennung erhält man 5-Phenylnikotinsäuremethylester und den entsprechenden, durch Umesterung entstandenen Isopropylester in einem Verhältnis von 2:3. Nach 48stündigem Rühren und ansonsten gleicher Reaktionsführung erhält man nur den Isopropylester.

Analog werden bei Verwendung der p-Alkylaryllithium-Verbindungen (hergestellt durch Metall-Halogenaustausch zwischen n-Butyllithium und p-Alkyl(brombenzolen) als Ausgangsverbindungen nach Abtrennen der durch Umesterung entstandenen Isopropylester folgende 5-Arylnikotinsäureester erhalten:

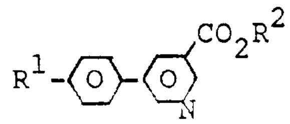

| $R^1$ | $R^2$ | Fp/°C |
|---|---|---|
| H | $CH_3$ | 45-47 |
| H | $C_2H_5$ (HCl) | 63-65 |
| H | $i$-$C_3H_7$ | |
| $CH_3$ | $CH_3$ | |
| $C_6H_{13}$ | $CH_3$ | |
| $H_{11}C_5$- 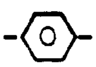 | $CH_3$ | |

Beispiel 8

Zu 14 ml einer 1,5 molaren Lösung von Phenyllithium in Benzol gibt man bei -78 °C 5,23 g Dicyclopentadienyltitandichlorid, gelöst in 20 ml Tetrahydrofuran, und rührt 1 Stunde bei -30 °C. Zu dieser Reaktionslösung werden nacheinander 50 mg 1,2-Bisdiphenylphosphinoethano-Nickelchlorid und 4,55 g 6-Bromnikotinsäuremethylester, gelöst in 20 ml Tetrahydrofuran gegeben, und 15 Stunden bei 60 °C gerührt. Nach üblicher Aufarbeitung erhält man 6-Phenylnikotinsäuremethylester.

Analog werden bei Verwendung der p-Alkylaryllithium-Verbindungen (hergestellt durch Metall-Halogenaustausch zwischen n-Butyllithium und p-Arylbrombenzolen) als Ausgangsmaterialien folgende 6-Aryl-Nikotinsäureester dargestellt:

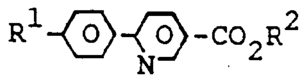

| $R^1$ | $R^2$ |
|---|---|
| H | $C_6H_{13}$ |
| H | 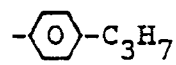 |
| $C_2H_5$ | $CH_3$ |
| $C_2H_5$ | $C_6H_{13}$ |
| $C_2H_5$ | 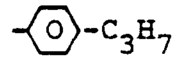 |
| $C_5H_{11}$ | $CH_3$ |
| $C_5H_{11}$ | $C_6H_{13}$ |
| $C_5H_{11}$ | 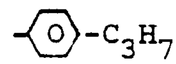 |
| $C_8H_{17}$ | $CH_3$ |
| $C_8H_{17}$ | $C_6H_{13}$ |
| $C_8H_{17}$ | 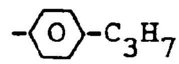 |
| 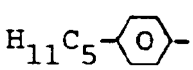 | $CH_3$ |
| 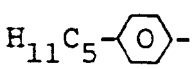 | $C_6H_{13}$ |

Analog erhält man mit 4-Alkylphenyl-magnesiumbromid oder 4-Alkylcyclohexylmagnesiumbromid und 2-(4-Bromphenyl)-5-Alkylpyrimidin 2-Biphenylylpyrimidine bzw. 2-(4-(4-Alkylcyclohexyl)-phenyl)-pyrimidine:

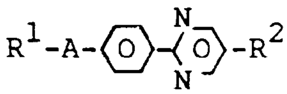

| $R^1$ | A | $R^2$ | |
|---|---|---|---|
| H | 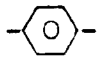 | H | |
| H | 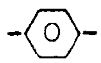 | $C_7H_{15}$ | K98 I |
| $C_5H_{11}$ | 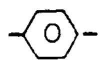 | $C_7H_{15}$ | K124 N168 I |
| $OC_3H_7$ | 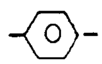 | $C_5H_{11}$ | K132 N204 I |
| $OC_5H_{11}$ | 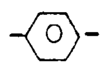 | $C_8H_{17}$ | |
| $C_5H_{11}$ | 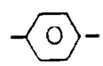 | 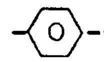-O-$C_3H_7$ | |
| $C_3H_7$ | 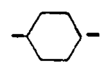 | $C_3H_7$ | K97 N198 I |

Analog erhält man aus optisch aktiven p-Alkoxyphenyllithium und 5-Alkyl-2-brompyrimidinen optisch aktive 2-(4-Alkoxyphenyl)-5-alkylpyrimidine.

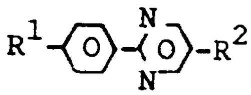

| | $R^1$ | $R^2$ | F (°C) | K (°C) |
|---|---|---|---|---|
| (R) | $CH_3$-CH(Cl)-CO-O | $C_9H_{19}$ | 69 | |
| | $C_2H_5$-$CHCH_3$-$CH_2O$ | $C_{11}H_{23}$ | 40 | 47 |
| | $C_2H_5$-$CHCH_3$-$(CH_2)_2$-O | $C_6H_{13}$ | 13 | 32 |
| | $C_2H_5$-$CHCH_3$-$(CH_2)_2$-O | $C_7H_{15}$ | 39 | |
| | $C_2H_5$-$CHCH_3$-$(CH_2)_2$-O | $C_8H_{17}$ | 36 | |
| | $C_2H_5$-$CHCH_3$-$(CH_2)_2$-O | $C_9H_{19}$ | 23 | 46 |
| | $C_2H_5$-$CHCH_3$-$(CH_2)_2$-O | $C_{10}H_{21}$ | 22 | 41 |
| | $C_2H_5$-$CHCH_3$-$(CH_2)_2$-O | $C_{11}H_{23}$ | 25 | 50 |
| | $C_2H_5$-$CHCH_3$-$(CH_2)_2$-O | $C_{12}H_{25}$ | 28 | 51 |
| | $C_2H_5$-$CHCH_3$-$(CH_2)_2$-O | $C_{11}H_{23}$ | 58 | 77 |
| | $C_2H_5$-$CH(CH_3)$-$(CH_2)_3$-O | $C_6H_{13}$ | 25 | 42 |
| | $C_2H_5$-$CH(CH_3)$-$(CH_2)_3$-O | $C_7H_{15}$ | 28,5 | 60 |
| | $C_2H_5$-$CH(CH_3)$-$(CH_2)_3$-O | $C_8H_{17}$ | 31 | 51 |
| | $C_2H_5$-$CH(CH_3)$-$(CH_2)_3$-O | $C_9H_{19}$ | 23 | 52 |
| | $C_2H_5$-$CH(CH_3)$-$(CH_2)_3$-O | $C_{10}H_{21}$ | 33 | 58 |
| | $C_2H_5$-$CH(CH_3)$-$(CH_2)_3$-O | $C_{11}H_{23}$ | 36 | 60 |
| | $C_2H_5$-$CH(CH_3)$-$(CH_2)_3$-O | $C_{12}H_{25}$ | 41 | 52 |
| | $C_2H_5$-$CH(CH_3)$-$CH_2O$-$(CH_2)_4$-O | $C_6H_{13}$ | -8 | 28 |
| | $C_2H_5$-$CH(CH_3)$-$CH_2O$-$(CH_2)_4$-O | $C_8H_{17}$ | 1 | 42 |
| | $C_2H_5$-$CH(CH_3)$-$CH_2O$-$(CH_2)_4$-O | $C_{10}H_{21}$ | 16 | 52 |
| | $C_8H_{17}O$ | -$(CH_2)_5$-$CHCH_3$-$C_2H_5$ | 14 | 44 |

Analog erhält man die entsprechenden optisch aktiven p-Alkylarylpyrazine aus 4-Alkylphenylmagnesiumbromid und 5-Alkyl-3-brompyrazinen:

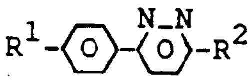

| $R^1$ | $R^2$ | F (°C) |
|---|---|---|
| $C_9H_{19}$ | $C_2H_5-CHCH_3-O$ | 78 |
| $C_5H_{11}$ | $C_2H_5-CHCH_3-O$ | 94 |
| $C_{12}H_{25}$ | $C_2H_5-CHCH_3-O$ | 78 |
| $C_5H_{11}$ | $CH_3-(CH_2)_5-CHCH_3-CH_2$ | 55 |
| $C_8H_{11}O$ | $CH_3-(CH_2)_5-CHCH_5-CH_2$ | 85 |

Beispiel 9

Aus 57,9 g 1-Brom-3,4-difluorbenzol, 4,2 g Lithium und 52,3 g Triisopropoxytitanchlorid in 750 ml Toluol/Tetrahydrofuran (4:1) erhält man gemäß Beispiel 2 mit Ultraschall die entsprechende Titanorganische Verbidnung. Zu diesem Reaktionsgemisch gibt man bei 0°-10 °C 86,6 g 1-Brom-4-jodbenzol und 4,4 g [1,1′-Bis-(diphenylphosphino)-ferrocenyl]-palladium-(II)-chlorid. Nach 48stündigem Rühren bei Raumtemperatur und üblichem Aufarbeiten erhält man 1-Brom-3′,4′-difluorbiphenyl.

Analog erhält man mit 1-Brom-3-fluor-4-jodbenzol als Ausgangsprodukt 1-Brom-3,3′,4′-trifluorbiphenyl.

Beispiel 10

Ein Gemisch aus 4-Brommagnesium-4′-pentylbiphenyl-1 (hergestellt aus 150 g 4-Brom-4′-pentylbiphenyl-1 und 10,8 g Magnesium) und 450 ml Tetrahydrofuran wird bei 5-20 °C mit einer Lösung von 118,8 g Triisopropoxytitanchlorid in 100 ml Tetrahydrofuran versetzt. Nach 30minütigem Rühren setzt man 5,85 g Bis-(triphenylphosphin)-nickel-II-chlorid und 83,7 g p-Brombenzonitril zu. Nach 16stündigem Rühren bei Raumtemperatur und üblicher Aufarbeitung erhält man 4-Cyano-4″-pentylterphenyl von Schmelzpunkt 130 °C und Klärpunkt 239 °C.

Analog erhält man
4-Cyano-4″-alkylterphenyle

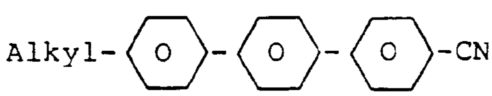

| Alkyl | K/N (°C) | N/I (°C) |
|---|---|---|
| $H_7C_3$ | 182 | 275 |
| $HgC_4$ | 154 | 242 |
| $H_{13}C_6$ | 125 | 228 |

**Patentansprüche**

1. Verfahren zur Herstellung von Verbindungen der Formel I,

$$R^1\text{-} A^o \text{-}(Z^1\text{-}A^1)_m\text{-}Z^o\text{-}(E)_n\text{-} Q \text{-}(Z^2\text{-}A^2)_p\text{-}R^2 \qquad I$$

worin

$R^1$ und $R^2$ jeweils unabhängig voneinander H oder Alkyl mit 1-18 C-Atomen, worin auch eine oder zwei nicht benachbarte $CH_2$-Gruppen durch -O-, -CO-, -O-CO-, -E-, -C≡C- und/ oder -CO-O- ersetzt sein können, CN oder Halogen

$A^o$, $A^1$ und $A^2$ jeweils unabhängig voneinander eine 1,4-Cyclohexylengruppe, worin auch eine oder zwei nicht benachbarte $CH_2$-Gruppen durch -O- und/oder -S- ersetzt sein können und/ oder die in 1-Stellung durch $R^3$ substituiert sein kann, eine 1,4-Cyclohexenylen-, eine Piperidin-1,4-diyl- oder 1,4-Bicyclo-[2,2,2]-octylengruppe, eine unsubstituierte oder ein- oder zweimal durch $R^3$, NC, NCO, und/oder NCS substituierte 1,4-Phenylengruppe, worin auch eine oder mehrere CH-Gruppen durch N ersetzt sein können, oder eine Pyridin-3,5-diylgruppe,

E $CR^3$=$CR^4$ oder $CHR^3$-$CHR^4$,

$R^3$, $R^4$ jeweils unabhängig voneinander H, Alkyl mit 1-6 C-Atomen, F, Cl, Br, $CF_3$, CN, mit der maßgabe, daß wenn E = $CHR^3$-$CHR^4$, $R^3$ und $R^4$ jeweils unabhängig voneinander Alkyl mit 1-6 C Atomen, F, Cl, Br, $CF_3$ bedeuten.

$Z^o$ eine Einfachbindung oder Alkylen mit 1-4 C-Atomen, worin auch eine oder zwei $CH_2$-Gruppen durch -CH(CN)-, -CH(F)- oder -CH(Cl)-Gruppen ersetzt sein können,

$Z^1$ und $Z^2$ jeweils -CO-O-, -O-CO-, -$CH_2CH_2$-, -CHCN-$CH_2$-, -$CH_2$-CHCN-, -CH=CH-, -$OCH_2$-, -$CH_2O$-, -CH=N-, -N=CH-, -NO-N-, -N=NO- oder eine Einfachbindung,

Q eine unsubstituierte oder ein- oder zweimal durch $R^3$ substituierte 1,4-Phenylengruppe, worin auch eine oder mehrere CH-Gruppen durch N ersetzt sein können, oder eine Pyridin-3,5-diylgruppe, oder eine 1,4-Cyclohexylengruppe, worin auch eine oder zwei nicht benachbarte $CH_2$-Gruppen durch -O- und-/oder -S- ersetzt sein können und/oder die in 1-Stellung durch $R^3$ substituiert sein kann, eine 1,4-Cyclohexenylen - oder 1,4-Bicyclo[2,2,2]-octylengruppe,

m und p jeweils 0, 1 oder 2,

n 0 oder 1 bedeuten,

dadurch gekennzeichnet, daß man eine Organotitanverbindung der Formel II,

$$R^1\text{-} A^o \text{-}(Z^1\text{-}A^1)_m\text{-}Z^o\text{-}Ti(Y)_3 \qquad II$$

worin $R^1$, $A^o$, $A^1$, $Z^o$, $Z^1$ und m die für Formel I angegebene Bedeutung besitzen und Y jeweils unabhängig voneinander Cl, Br, $R^5$, O-$R^5$, $NR^5_2$ oder ein Rest $R^1$- $A^o$ -($Z^1$-$A^1$)$_m$-$Z^0$- mit der voranstehenden Bedeutung ist, wobei $R^5$ verzweigtes oder unverzweigtes Alkyl mit 1-6 C-Atomen oder Cycloalkyl mit 3-7 -C-Atomen bedeutet, mit einer Verbindung der Formel III,

$$X\text{-}(E)_n\text{-} Q \text{-}(Z^2\text{-}A^2)_p\text{-}R^2 \qquad III$$

worin $R^2$, E, $A^2$, $Z^2$, Q, n und p die für Formel I angegebene Bedeutung besitzen und X Br, J, $OSO_2$-$(CF_2)_r$-$CF_3$, worin r 0, 1, 2, 3, 4, 5, 6, 7, 8, 9 oder 10 ist, bedeutet, unter Zusatz einer Verbindung des Palladiums und/oder Nickels koppelt.

2. Verfahren nach Anspruch 1, dadurch gekennzeichnet, daß man zur Herstellung von Verbindungen der Formel II Verbindungen der Formel IV,

$$R^1\text{-} A^o \text{-}(Z^1\text{-}A^1)_m\text{-}Z^o\text{-}X' \qquad IV$$

worin $R^1$, $A^o$, $A^1$, $Z^o$, $Z^1$ und m die für Formel I angegebene Bedeutung besitzen und X' Chlor, Brom, Iod bedeutet, in einem inerten Lösungsmittel mit metalischem Lithium, n-Butyllithium, tert-Butyllithium, Lithiumnaphthalenid, Lithium-di-tert-Butylnaphthalenid oder metalischem Magnesium umsetzt und anschließend eine Titanverbindung der Formel V

$$Ti(Y')_4 \qquad V$$

worin Y' jeweils unabhängig voneinander Cl, Br, $R^5$, $OR^5$ oder $NR^5_2$ bedeutet, wobei $R^5$die angegebene Bedeutung besitzt, zusetz.

3. Verfahren nach Anspruch 2, dadurch gekennzeichnet, daß man Verbindungen ausgewählt aus den Formeln IVb, IVd, IVe, IVg, IVh, IVi, IVk

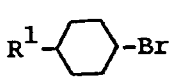

IVb

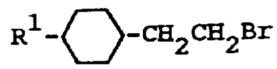

IVd

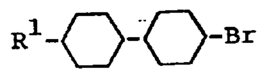

IVe

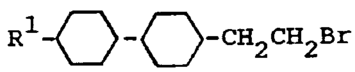

IVg

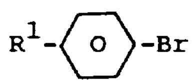

IVh

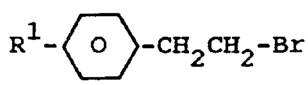

IVi

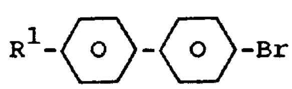

IVk

worin $R^1$ die angegebene Bedeutung besitzt, in der angegebenen Weise umsetzt.

**4.** Verfahren nach Anspruch 2, dadurch gekennzeichnet, daß man Tianverbindungen ausgewählt aus den Formeln Va bis Vn verwendet:

| | |
|---|---|
| $Cl_3Ti(OR^5)$ | Va |
| $Cl_3Ti(N^5_2)$ | Vb |
| $Br_3Ti(OR^5)$ | Vc |
| $Br_3Ti(NR^5_2)$ | Vd |
| $Cl_2Ti(OR^5)_2$ | Ve |
| $Cl_2Ti(NR^5_2)_2$ | Vf |
| $Br_2Ti(OR^5)_2$ | Vg |
| $Br_2Ti(NR^5_2)_2$ | Vh |
| $ClTi(OR^5)_3$ | Vi |
| $ClTi(NR^5_2)_3$ | Vj |
| $BrTi(OR^5)_3$ | Vk |
| $BrTi(NR^5_2)_3$ | Vl |
| $Cl_2Ti(R^5)_2$ | Vm |
| $Br_2Ti(R^5)_2$ | Vn |

worin $R^5$ eine Alkylgruppe mit 1-3 C-Atomen bedeutet.

**5.** Verfahren nach Anspruch 1, dadurch gekennzeichnet, daß man die Kopplung in Gegenwart von Tetrakis-(triphenylphosphin)-palladium oder von [1,1′-Bis-(Diphenylphosphin)ferrocenyl]-palladium durchführt.

## Claims

1. Process for the preparation of compounds of the formula I

$$R^1\text{-}A^0\text{-}(Z^1\text{-}A^1)_m\text{-}Z^0\text{-}(E)_n\text{-}Q\text{-}(Z^2\text{-}A^2)_p\text{-}R^2 \qquad \text{I}$$

wherein

$R^1$ and $R^2$ in each case independently of one another are H or alkyl having 1-18 C atoms, wherein one or two non-adjacent $CH_2$ groups can also be replaced by -O-, -CO-, -O-CO-, -E-, -C≡C- and/or -CO-O-, CN or halogen,

$A^0$, $A^1$ and $A^2$ in each case independently of one another are a 1,4-cyclohexylene group, wherein one or two non-adjacent $CH_2$ groups can also be replaced by -O- and /or -S- and/or which can be substituted in the 1-position by $R^3$, or a 1,4-cyclohexenylene, a piperidine-1,4-diyl or 1,4-bicyclo-[2,2,2]-octylene group, a 1,4-phenylene group which is unsubstituted or mono- or disubstituted by $R^3$, NC, NCO and/or NCS and wherein one or more CH groups can also be replaced by N, or a pyridine-3,5-diyl group,

E is $CR^3$=$CR^4$ or $CHR^3$-$CHR^4$,

$R^3$ and $R^4$ in each case independently of one another are H, alkyl having 1-6 C atoms, F, Cl, Br, $CF_3$ or CN, with the proviso that if E = $CHR^3$-$CHR^4$, $R^3$ and $R^4$ in each case independently of one another are alkyl having 1-6 C atoms, F, Cl, Br or $CF_3$.

$Z^0$ is a single bond or alkylene having 1-4 C atoms, wherein one or two $CH_2$ groups can also be replaced by -CH(CN)-, -CH(F)- or -CH(Cl)- groups,

$Z^1$ and $Z^2$ are each -CO-O-, -O-CO-, -$CH_2$ $CH_2$-, -CHCN-$CH_2$-, -$CH_2$-CHCN-, -CH=CH-, -$OCH_2$-, -$CH_2O$-, -CH=N-, -N=CH-, -NO=N-, -N=NO- or a single bond,

Q is a 1,4-phenylene group which is unsubstituted or mono- or disubstituted by $R^3$ and wherein one or more CH groups can also be replaced by N, or a pyridine-3,5-diyl group,

or a 1, 4 -cyclohexylene group, wherein one or two non-adjacent $CH_2$ groups can also be replaced by -O- and/or -S- and/or which can be substituted in the 1-position by $R^3$, or a 1,4-cyclohexenylene or 1,4-bicyclo-[2,2,2]-octylene group,

m and p are each 0, 1 or 2 and

n is 0 or 1,

characterised in that an organotitanium compound of the formula II

$$R^1\text{-}A^0\text{-}(Z^1\text{-}A^1)_m\text{-}Z^0\text{-}Ti(Y)_3 \qquad \text{II}$$

wherein $R^1$, $A^0$, $A^1$, $Z^0$ $Z^1$ and m have the meaning given in the case of formula I and the radicals Y in each case independently of one another are Cl, Br, $R^5$, O-$R^5$, $NR^5_2$ or a radical $R^1$-$A^0$-($Z^1$-$A^1$)$_m$-$Z^0$- with the above meaning, wherein $R^5$ is branched or unbranched alkyl having 1-6 C atoms or cycloalkyl having 3-7 C atoms, is coupled with a compound of the formula III

$$X\text{-}(E)_n\text{-}Q\text{-}(Z^2\text{-}A^2)_p\text{-}R^2 \qquad \text{III}$$

wherein $R^2$, E, $A^2$, $Z^2$, Q n and p have the meaning given in the case of formula I and X is Br, I or $OSO_2$-$(CF_2)_r$-$CF_3$, wherein r is 0, 1, 2, 3, 4, 5, 6, 7, 8, 9 or 10, with addition of a palladium and/or of nickel compound.

2. Process according to Claim 1, characterised in that, to prepare compounds of the formula II, compounds of the formula IV

$$R^1\text{-}A^0\text{-}(Z^1\text{-}A^1)_m\text{-}Z^0\text{-}X' \qquad \text{IV}$$

wherein $R^1$, $A^0$, $A^1$, $Z^0$, $Z^1$ and m have the meaning given in the case of formula I and X' is chlorine, bromine or iodine, are reacted with metallic lithium, n-butyllithium, tert-butyllithium, lithium naphthalenide, lithium di-tert-butylnaphthalenide or metallic magnesium in an inert solvent, and then a titanium compound of the formula V

$$Ti(Y')_4 \qquad \text{V}$$

wherein the radicals Y' in each case independently of one another are Cl, Br, $R^5$, $OR^5$ or $NR^5_2$, wherein $R^5$ has the meaning given, is added.

3. Process according to Claim 2, characterised in that compounds chosen from the formulae IVb, IVd, IVe, IVg, IVh, IVi, IVk

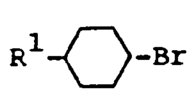 IVb

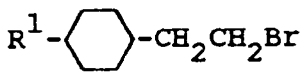

IVd

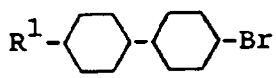

IVe

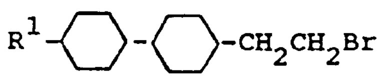

IVg

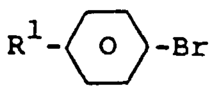

IVh

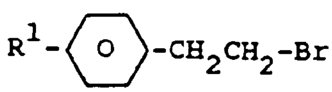

IVi

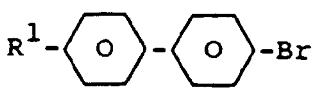

IVk

wherein $R^1$ has the meaning given, are reacted in the manner described.

**4.** Process according to Claim 2, characterised in that titanium compounds chosen from the formulae Va to Vn are used:

| | |
|---|---|
| $Cl_3Ti(OR^5)$ | Va |
| $Cl_3Ti(NR^5_2)$ | Vb |
| $Br_3Ti(OR^5)$ | Vc |
| $Br_3Ti(NR^5_2)$ | Vd |
| $Cl_2Ti(OR^5)_2$ | Ve |
| $Cl_2Ti(NR^5_2)_2$ | Vf |
| $Br_2Ti(OR^5)_2$ | Vg |
| $Br_2Ti(NR^5_2)_2$ | Vh |
| $ClTi(OR^5)_3$ | Vi |
| $ClTi(NR^5_2)_3$ | Vj |
| $BrTi(OR^5)_3$ | Vk |
| $BrTi(NR^5_2)_3$ | Vl |
| $Cl_2Ti(R^5)_2$ | Vm |
| $Br_2Ti(R^5)_2$ | Vn |

wherein $R^5$ is an alkyl group having 1-3 C atoms.

**5.** Process according to Claim 1, characterised in that the coupling is carried out in the presence of tetrakis(triphenylphosphine)palladium or of [1,1′-bis-(diphenylphosphine)ferrocenyl]palladium.

## Revendications

**1.** Procédé de préparation des composés de formule I :

$$R^1\text{-}A^0\text{-}(Z^1\text{-}A^1)_m\text{-}Z^0\text{-}(E)_n\text{-}Q\text{-}(Z^2\text{-}A^2)_p\text{-}R^2 \qquad (I)$$

dans laquelle :

$R^1$ et $R^2$ représentent chacun, indépendamment l'un de l'autre, l'hydrogène ou un groupe alkyle en $C_1$-$C_{18}$ dans lequel un ou deux groupes $CH_2$ non voisins peuvent également être remplacés par -O-, -CO-, -O-CO-, -E-, -C≡C- et/ou -CO-O-, un groupe CN ou un halogène,

$A^0$ , $A^1$ et $A^2$ représentent chacun, indépendamment les uns des autres, un groupe 1,4-cyclohexylene dans lequel un ou deux groupes $CH_2$ non voisins peuvent être également remplacés par -O- et/ou -S- et/ou qui peut être substitué en position 1 par $R^3$, un groupe 1,4-cyclohexénylène, pipéridine-1,4-diyle ou 1,4-bicyclo[2,2,2] octylène, un groupe 1,4-phénylène non substitué ou mono- ou di-substitué par $R^3$, NC, NCO et/ou NCS et dans lequel un ou plusieurs groupes CH peuvent également être remplacés par N, ou un groupe pyridine-3,5-diyle,

E représente $CR^3$=$CR^4$ ou $CHR^3$-$CHR^4$,

$R^3$ et $R^4$ représentent chacun, indépendamment l'un de l'autre, l'hydrogène, un groupe alkyle en $C_1$ -$C_6$, F, Cl, Br, CF3, CN, sous réserve que lorsque E représente $CHR^3$-$CHR^4$, $R^3$ et $R^4$ représentent chacun, indépendamment l'un de l'autre, un groupe alkyle en $C_1$ -$C_6$, F, Cl, Br, $CF_3$,

$Z^0$ représente une liaison simple ou un groupe alkylène en $C_1$-$C_4$ dans lequel un ou deux groupes $CH_2$ peuvent également être remplacés par des groupes -CH(CN)-, -CH(F)-ou -CH(Cl)-,

$Z^1$ et $Z^2$ représentent chacun -CO-O-, -O-CO-, -$CH_2CH_2$-, -CHCN-$CH_2$-, -$CH_2$CHCN-, -CH=CH-, -$OCH_2$-, -$CH_2$O-, -CH=N-, -N=CH-, -NO=N-, -N=NO- ou une liaison simple,

Q représente un groupe 1,4-phénylène non substitué ou mono- ou di-substitué par $R^3$ et dans lequel un ou plusieurs groupes CH peuvent également être remplacés par N, ou un groupe pyridine-3,5-diyle,

ou un groupe 1,4-cyclohexylene dans lequel un ou deux groupes $CH_2$ non voisins peuvent également être remplacés par -O- et/ou -S- et/ou qui peut être substitué en position 1 par $R^3$, un groupe 1,4-cyclohexénylène ou 1,4-bicyclo [2,2,2]octylène,

m et p sont chacun égaux à 0, 1 ou 2,

n est égal à 0 ou 1,

caractérisé en ce que l'on couple un dérivé d'organo-titane de formule II :

$$R^1\text{-}A^0\text{-}(Z^1\text{-}A^1)_m\text{-}Z^0\text{-}Ti(Y)_3 \qquad (II)$$

dans laquelle $R^1$, $A^0$, $A^1$, $Z^0$, $Z^1$ et m ont les significations indiquées en référence à la formule I, et chacun des symboles Y représente, indépendamment des autres, Cl, Br, $R^5$, O-$R^5$, $NR^5_2$ ou un groupe $R^1$-$A^0$-($Z^1$-$A^1$)$_m$-$Z^0$- dont la signification a été donnée ci-dessus, $R^5$ représentant un groupe alkyle à chaîne droite ou ramifiée en $C_1$-$C_6$ ou cycloalkyle en $C_3$-$C_7$, avec un composé de formule III :

$$X\text{-}(E)_n\text{-}Q\text{-}(Z^2\text{-}A^2)_p\text{-}R^2 \qquad (III)$$

dans laquelle $R^2$, E, $A^2$, $Z^2$, Q, n et p ont les significations indiquées en référence à la formule I et X représente Br, I, un groupe $OSO_2$-$(CF_2)_r CF_3$ dans lequel r est égal à 0, 1, 2, 3, 4, 5, 6, 7, 8, 9 ou 10, en présence d'un composé du palladium et/ou du nickel.

**2.** Procédé selon la revendication 1, caractérisé en ce que, pour la préparation des composés de formule II, on fait réagir des composés de formule IV :

$$R^1\text{-}A^0\text{-}(Z^1\text{-}A^1)_m\text{-}Z^0\text{-}X' \qquad (IV)$$

dans laquelle $R^1$, $A^0$, $A^1$, $Z^0$, $Z^1$ et m ont les significations indiquées en référence à la formule I, et X' représente le chlore, le brome, l'iode, dans un solvant inerte avec du lithium métallique, du n-butyllithium, du tert.-butyllithium, du naphtalénure de lithium, du di-tert.-butylnaphtalénure de lithium ou du magnésium métallique puis on ajoute un dérivé du titane de formule V :

$$Ti(Y')_4 \qquad (V)$$

dans laquelle chacun des symboles Y' représentent, indépendamment des autres Cl, Br, $R^5$, $OR^5$ ou $NR^5_2$ danslesquels $R^5$ a les significations indiquées ci-dessus.

**3.** Procédé selon la revendication 2, caractérisé en ce que l'on fait réagir de la manière indiquée des composés choisis parmi ceux qui répondent aux formules IVb, IVd, IVe, IVg, IVh, IVi et IVk :

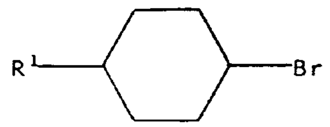

(IVb)

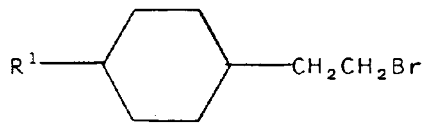

(IVd)

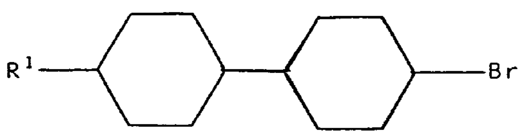

(IVe)

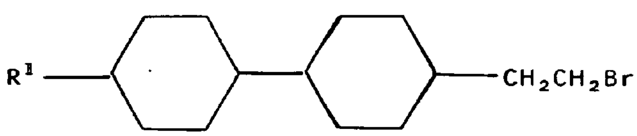

(IVg)

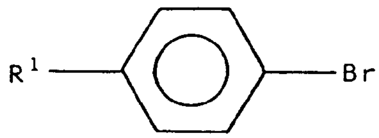

(IVh)

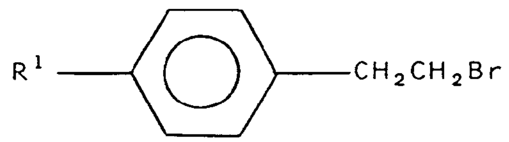

(IVi)

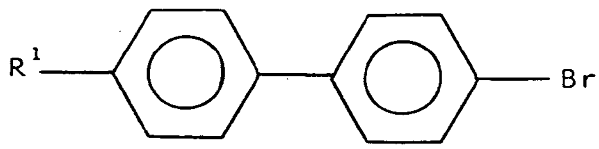

(IVk)

dans lesquelles R [1] a les significations indiquées ci-dessus.

4. Procédé selon la revendication 2, caractérisé en ce que l'on choisit les dérivés du titane parmi ceux qui répondent aux formules Va à Vn :

| | |
|---|---|
| $Cl_3Ti(OR^5)$ | Va |
| $Cl_3Ti(NR^5_2)$ | Vb |
| $Br_3Ti(OR^5)$ | Vc |
| $Br_3Ti(NR^5_2)$ | Vd |
| $Cl_2Ti(OR^5)_2$ | Ve |
| $Cl_2Ti(NR^5_2)_2$ | Vf |
| $Br_2Ti(OR^5)_2$ | Vg |
| $Br_2Ti(NR^5_2)_2$ | Vh |
| $ClTi(OR^5)_3$ | Vi |
| $ClTi(NR^5_2)_3$ | Vj |
| $BrTi(OR^5)_3$ | Vk |
| $BrTi(NR^5_2)_3$ | Vl |
| $Cl_2Ti(R^5)_2$ | Vm |
| $Br_2Ti(R^5)_2$ | Vn |

dans lesquelles $R^5$ représente un groupe alkyle en $C_1$-$C_3$.

5. Procédé selon la revendication 1, caractérisé en ce que l'on procède au couplage en présence du tétrakis-(triphénylphosphine(-palladium ou du [1,1'-bis-(dlphényl-phosphine)-ferrocényl]-palladium.